# EUROPEAN PATENT APPLICATION

(11) **EP 2 757 543 A1**
(43) Date of publication of application: **23.07.2014**
(21) Application number: 11872401.2
(22) Date of filing: 13.09.2011
(51) Int. Cl.: G09B 21/00, A61F 9/08

(54) **PORTABLE ELECTRONIC BRAILLE READER**

(71) Applicant: Rodriguez Regalado, Mauricio, Guadalajara, Jalisco 44690 (MX)
(72) Inventor: Rodriguez Regalado, Mauricio, Guadalajara, Jalisco 44690 (MX)
(74) Representative: ZBM Patents - Zea, Barlocci & Markvardsen
(86) International application number: PCT/MX2011/000106
(87) International publication number: WO 2013/039369

(57) **Abstract**

A portable electronic Braille reader comprising a capacitive screen to which information is inputted through an input port or any other appropriate means, said information being decoded and encoded by a microprocessor to convert it into Braille characters; a thimble that includes elements capable of generating a stimulus in the finger of the user so the user recognizes the characters represented on the screen, and a line guide to ensure that the thimble slides in the correct direction and paths during reading.

## Description

### FIELD OF THE INVENTION

The invention relates to electronic readers, and more particularly to a portable electronic Braille reader that allows visually impaired or blind people to "read" any printed material that is incorporated to such reader by any suitable means, known or not yet known.

### BACKGROUND OF THE INVENTION

Spanish patent ES 2202570 describes a portable reading device for the blind that integrates optical sensors capable of processing printed text, an electronic system that stores software adapted to recognize printed characters as well as conversion software of such characters into Braille characters, a touch recognition area and a microcapsule attached to a sheath into which the tip of the blind person's index finger is introduced, much like a thimble. The microcapsule contains an optical sensor array provided with a lens and an electromagnetic unit designed to display Braille character by character on a touch surface, as soon as the blind person's index finger is moved over a printed character and when the finger is exactly on that character. Such electromagnetic unit comprises six magnets intended to reproduce Braille characters and two more intended to inform the reader of a mistake in the direction; the latter are activated if the blind person deviates his/her index finger upwards or downwards during its displacement along a line of text.

One difference between the reading device of the aforementioned Spanish patent and the new reader, is that the latter does not use an optical character recognition (OCR) procedure to input in the reading device the text that has to be made available to the blind person. In the new reader, the information to be loaded is electronic files that are converted to Braille text. Therefore, the reader of the present invention does not require optical sensors, nor the software associated to that procedure of recognition, and the entry of data to the new reader can be done through information storage means (USB stick, CD, DVD) or via internet, as described later. Logically, the latter procedure is much more versatile, reliable and direct than the use of optical sensors.

Another important difference between the Spanish patent and the new reader is that the reading device of the former does not have a receptive screen, while the new reader does. Another substantial difference is that the new reader has the ability to receive information via WiFi in the future, while the device of the Spanish patent does not.

Another noticeable difference is that the cell in the Braille thimble of the new reader can be actuated by electromagnets or by so called "muscle wire". Because the source of the text is electronic files, with the new reader the problem of the Spanish patent regarding how text of different sizes is read, or which is the distance between the text and the thimble, does not arise.

### OBJECTS OF THE INVENTION

The main object of the invention is to propose a new portable electronic Braille reader characterized in that it comprises a capacitive screen, which consists of a screen that by means of direct touch on its surface allows entry of data and commands to the portable electronic reader; the information to be read is inputted to the screen through an input port or any other suitable means. Such information is decoded and encoded by a microprocessor to convert it into Braille characters that can be recognized by a thimble, which is placed on the index finger of a blind person and which for this purpose includes a Braille cell with elements capable of generating stimulus on the finger of the reader so the person can recognize the Braille characters that correspond to specific positions on the screen; and a line guide to ensure that the thimble slides in the correct direction and paths during reading.

The capacitive screen operates such that when the reader presses on an X, Y coordinate, this coordinate sends a signal to the thimble and indicates that the Braille cell must raise and lower the points of the cell to form a letter. When moving to the right one position, that is the X +1, Y coordinate, the screen sends electronic impulses to report that the position corresponds to another letter in Braille, and this causes the thimble to raise or lower the points of the Braille cell to form the new letter of said position.

### BRIEF DESCRIPTION OF THE FIGURES OF THE INVENTION

Figure 1 is a schematic view showing the main components of the portable electronic Braille reader of the present invention.
Figure 2 is a view of the reader's capacitive screen which contains the information that was previously stored therein for reading.
Figure 3 is a perspective view of an exemplary embodiment of the thimble which is an essential element of the reader of the present invention.
Figure 4 is a view showing the components of the Braille cell that is housed in the thimble of the electronic reader.
Figure 5 shows the relationship between the capacitive screen and the thimble of the electronic Braille reader.
Figure 6 shows that the thimble has a notch that prevents it from being positioned over the receptive screen if it is not aligned with the lower guide. This ensures that when reading is started, the person always has the portable electronic Braille reader in the right direction.
Figure 7 is illustrative of the guide system used to prevent the electronic reader from leaving the row that is being read.
Figure 8 is an exemplary model of an embodiment of the assembled portable electronic Braille reader of the present invention.

### DETAILED DESCRIPTION OF THE PREFERRED EMBODIMENT OF THE INVENTION

An electronic Braille reader to allow blind people to access printed material such as books, magazines and newspapers, which allows the blind to read a whole page in Braille. The reading device of the present invention is portable and by accessing the internet or inserting a USB, CD or DVD, or any other known or not yet known format, is loaded with files that are decoded for reading.

The main parts that make up the electronic reader (100) are:
- Upper Housing (10)
- Lower Housing (20)
- Battery (30)
- USB Connector (40)
- Microprocessor (PCBA) (50)
- Action Buttons (60, 62, 64)
- Capacitive Screen (70)
- Thimble (80)
- Line Guide (90)

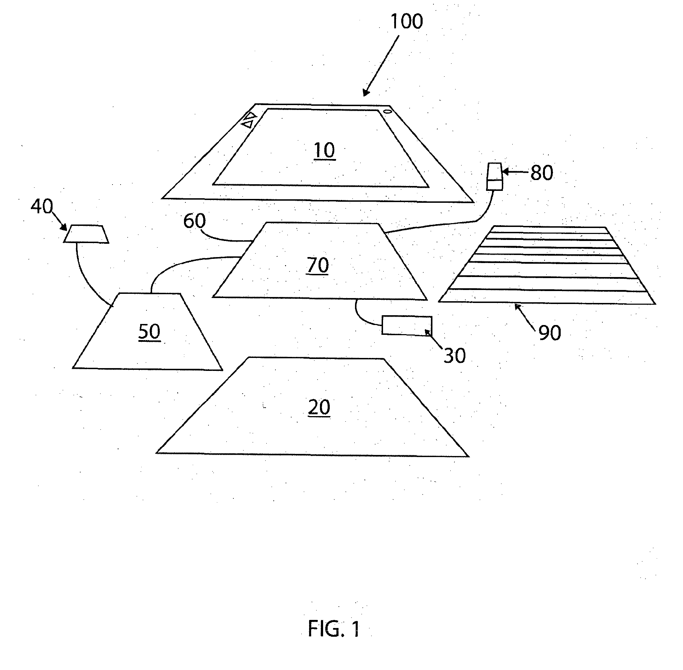

Figure 1 shows the electronic reading device (100) in disassembled condition to clearly reveal the main elements in it, wherein the lower housing (20) and the upper housing (10) aim to provide protection and union to the USB connector (40), the action buttons (60), the microprocessor (50), which may be a printed circuit board PCBA, the capacitive screen (70), the battery (30) and the line guide (90). The capacitive screen can be like the ones used by other devices commonly known as "touch screen", i.e. iPad or iPhones, where certain commands are executed by direct touch on the screen surface.

The power source of the electronic reader (100) may be a rechargeable lithium-iron-phosphate battery (30) whose advantage over lithium-phosphate (commonly used in cell phones and laptops) is that they are non-toxic to the environment, do not explode, do not corrode or suffer damages due to high fluctuations in ambient temperature and have a shelf life of 5-6 years.

The USB connector (40) is the input port of the information that will be accessible to the blind person. Such information is received by the USB connector (40) and taken to the printed circuit board (PCBA) (50) to be decoded. The PCBA will be adapted to control the functions of the reader. Powered by the battery (30), the PCBA receives the files from the USB connector (40), decodes them and encodes them so they can be read by the capacitive screen (70). The PCBA (50) also receives signals from the action buttons (60, 62, 64), which allows to turn on/off the electronic reader (100) and go page forward/back. The source file that is inputted in the reading device can be in txt, doc, pdf or any other format. This file source is inputted through the USB port and a specially developed software that is built into the processor translates each letter of the inputted text to a specific coordinate on the screen (70).

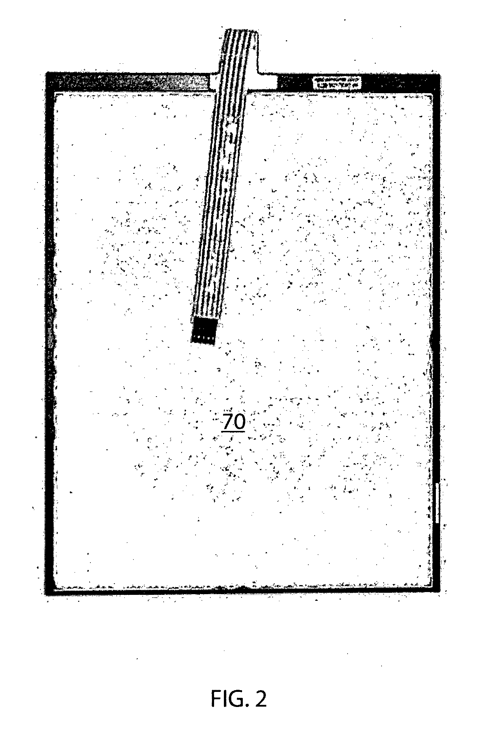

The capacitive screen (70) is a touch screen (Fig. 2) which by direct touch on its surface allows the input of data and commands to the portable electronic reader. After receiving the information from the PCBA, the capacitive screen (70) contains information equivalent to a written page. The thimble (80) is connected to the circuit board (PCBA), and by placing the thimble on a particular point of the capacitive screen, the latter recognizes the coordinate at which the thimble (80) is positioned and determines which Braille character corresponds to that particular coordinate and sends a signal to the thimble to raise and lower the relevant points of the Braille cell. Each character that make up the words is associated with an X,Y coordinate on the screen. The screen shows nothing, it is blank. What the screen does is that when the reader presses on the X,Y coordinate, this coordinate will send a signal to the thimble and indicate that the Braille cell must raise and lower the points of the cell to form a letter. When moving on a position on the right, the X +1,Y coordinate, the screen sends electronic impulses to report that said location corresponds to another letter in Braille, and this causes the thimble to lower and raise the points of the Braille cell to form the new letter corresponding to that new position on the screen.

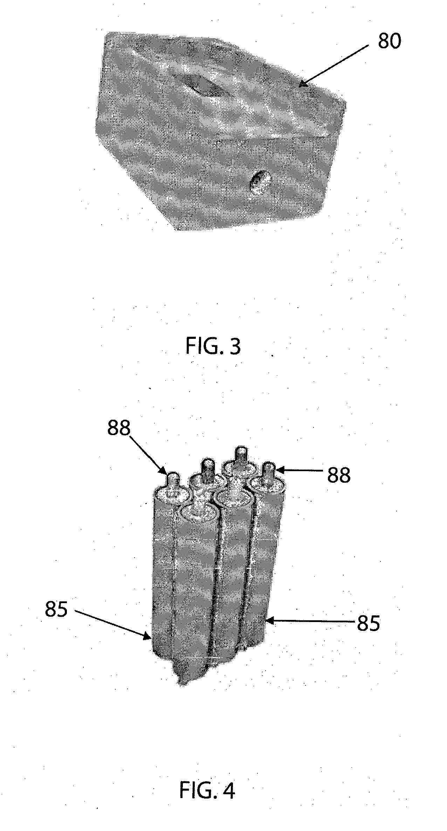

Another component of the portable electronic Braille reader is the thimble (80), shown as an example in Figure 3, which is a piece that is placed on the tip of the index finger of the blind person and is adapted to be secured to the finger by any appropriate means. The thimble (80) is operatively connected to the PCBA (50) via a cable that allows it to receive signals from the PCBA. It is understood that it may also be implemented whatever is necessary for the connection between the thimble (80) and the PCBA (50) to be wireless.

Figure 4 shows a Braille cell that is housed in the thimble and which consists of a set of electromagnets (85) that are arranged in an array of two columns of three electromagnets each. Each electromagnet (85) houses in its center an iron cylinder (88) of approximately 1.5 mm in diameter, which when subjected to the electromagnetic force of the electromagnets can be lifted from its initial rest position to a height of approximately 0.5 mm.

The electromagnets (85) operate independently of one another, so that the combination of raising and holding down of the cylinders (88) allows for Braille characters to be formed, based on the information inputted into the reader for its reading.

When the thimble (80) is placed on a particular point of the capacitive screen, the latter detects its position in a X,Y coordinate system and sends the signal such that the thimble, which contains the Braille cell, raises the points equivalent to the letter that corresponds to that position on the screen. A Braille cell consists of six or eight points that allow displaying Braille characters. These points, unlike printed Braille, can alternate between raised and lowered positions, so they can vary dynamically.

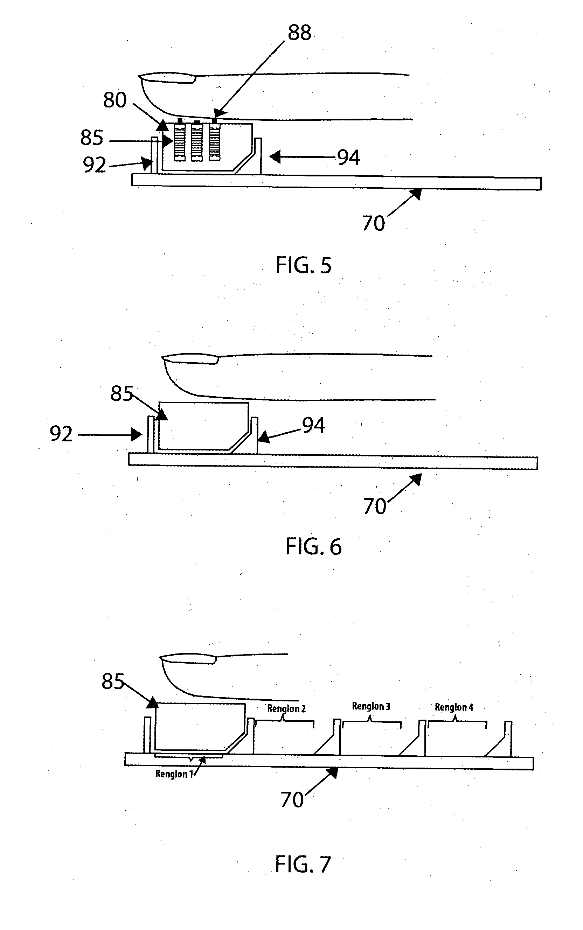

Figure 5 is a representation illustrating the use of the electronic reader (100), which shows how the thimble (80) is placed on the receptive screen and slid laterally as it reads the data that has been previously inputted to the reader. To maintain a linear trajectory while sliding the thimble through the rows of the screen, a line guide (90) is provided, which consists of a grid is placed over the capacitive screen (70). The line guide (90) indicates to the blind reader the correct direction of the page, and makes sure that the blind reader stays on the row to be read.

The line guide (90) comprises an upper guide (92) and a lower guide (94) which together define a path or passage through which the thimble (80) is made to slide as it reads the information on the capacitive screen. The thimble (80) has a notch (88) or recessed section which prevents the thimble from being positioned over the receptive screen if it is not aligned with the complementary recessed surface (95) provided on the lower guide (94). This ensures that to start reading the user always has the electronic reader in the correct position, and therefore in the correct reading direction.

Another function of the guide (90) is to prevent the reader from departing from the row it is reading. The upper guide (92) and the lower guide (94) confine the path, from one end to the other end of the screen, that the reader must follow while reading. Once the reader has finished reading a row, the thimble (80) is placed on the left edge of the next lower row, and so on, until all the rows of the page have been read (Fig. 7).

As shown in figure 5, the cylinders (86) inserted in the electromagnets (85), adapted to move up and down, have a different elevation relationship to each other as a result of the electromagnetic force exerted on them because of the Braille character corresponding to a particular position on the screen (70), which depends on the information inputted into the electronic reader. Thus, the reader will perceive in his index finger the character or letter which is in that particular position of the screen.

Once all rows on the receptive screen (70) have been read, the blind reader press the action button (64) provided in the electronic reading device (100) to advance to the next page. The PCBA (50) receives the corresponding command and provides new information to the screen so it can be read by the user. At the same time, the reader places the thimble on the left edge of the first row and repeats the reading process as done in the previous page. In the event that the user has a doubt about previously read content, the user can press the action button (62) to move the page back, and the PCBA (50) provides the capacitive screen with the previously accessed information.

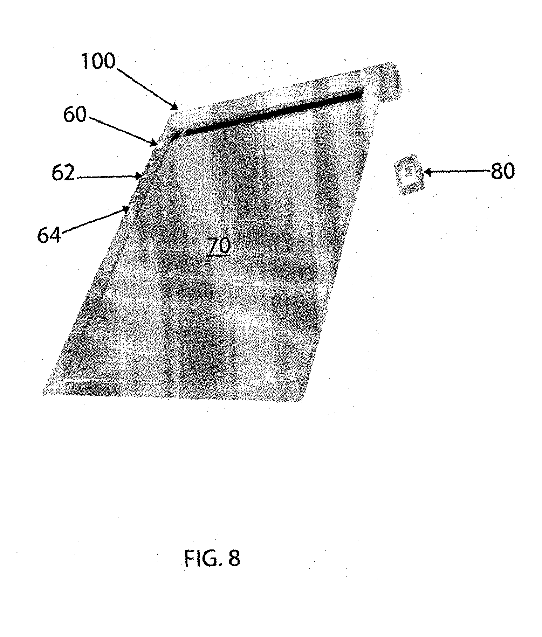

Figure 8 shows an assembled exemplary model of the portable electronic Braille reader (100) previously described. It is to be understood that the embodiment of the reader can be similar or different from the one illustrated in this figure and that this does not imply that it differs or diverts from the structural and functional concept as described hereinabove.

Although the invention has been described in the context of preferred embodiments, it will be apparent to those skilled in the art that the scope of the concept extends beyond the specifically disclosed model to other possible embodiments of the invention that are obvious and deducible from the contents of the foregoing description. Moreover, while the invention has been described in detail, one skilled in the art to which the invention pertains may deduce that some identified components can be replaced by others different or similar.

Given the above, it is understood that one or more of the primary or secondary elements of the electronic reader can be combined with others, or replaced by others to embody a reader with the previously described features. Accordingly, it is intended that the scope of the present invention is not interpreted in terms of the particular embodiments disclosed, but it is determined by reasonable interpretation of the content of the following claims.

It is noted that the best way to implement the present invention is that which is derived from the content of the foregoing description.

## Claims

1. Portable electronic Braille reader, comprising:
- a protective housing, which contains:
a) a capacitive screen capable of receiving and storing information via any suitable means and making it available to a blind person, to be read in Braille; said screen being further adapted to input data or commands by touch;
b) means for inputting information to the electronic reader;
c) a microprocessor for decoding the information inputted to the reader and encoding it so it can be read by capacitive screen;
d) action buttons through which certain signals are sent to the microprocessor to turn on/off the electronic reader and move pages forward/back;
e) a power supply as to provide the reader with power;
f) a line guide to ensure that the thimble slides over the rows in the correct direction and follows a linear path from one end to the other end of the screen;
- a thimble connected to the microprocessor and adapted to be slid over the capacitive screen, as to allow a user wearing it to perceive the characters representing the decoded and coded information that was previously inputted into the reader; said thimble including a Braille cell comprising magnetic elements that generate an electromagnetic force, each having a cylinder inserted therein, wherein said cylinders can be raised independently from each other, to enable the blind person to perceive, in a specific position of the thimble on the capacitive screen, a certain Braille character.

2. The electronic reader of claim 1, wherein the line guide extend from one end to other end of the screen along the trajectory of a reading row.

3. The electronic reader of claim 1, wherein the thimble and the line guide are configured to engage in a complementary way, whereby the correct positioning of said thimble on the receptive screen is ensured.

4. The electronic reader of claim 1, wherein the line guide consist of a grid which is placed on the capacitive screen to define the paths to be followed by the thimble during the reading process.

5. The electronic reader of claim 1, wherein the means for inputting information to the reader is an input port.

6. The electronic reader of claim 1, wherein the information is inputted to the reader through a USB, a CD, DVD or any other medium for storage of computer readable information.

7. The electronic reader of claim 1, wherein reading information is inputted into the reader by downloading it from the internet.

8. The electronic reader of claim 1, wherein the line guide and the thimble together define a means for ensuring that said thimble is correctly positioned on the receptive screen.

9. The electronic reader of claim 1, wherein communication between the microprocessor and the thimble can be wired or wireless.

10. The electronic reader of claim 1, wherein each character forming the words is associated with an X,Y coordinate on the screen.
